# EUROPEAN PATENT SPECIFICATION

(11) **EP 4 045 487 B1**
(45) Date of publication and mention of the grant of the patent: **12.02.2025**
(21) Application number: 20804379.4
(22) Date of filing: 16.10.2020
(51) Int. Cl.: C07D 231/16, C07D 401/04, C07D 405/04

(54) **METHODS FOR THE PREPARATION OF 5-BROMO-2-(3-CHLORO-PYRIDIN-2-YL)-2H-PYRAZOLE-3-CARBOXYLIC ACID**
VERFAHREN ZUR HERSTELLUNG VON 5-BROM-2-(3-CHLOR-PYRIDIN-2-YL)-2H-PYRAZOL-3-CARBONSÄURE
PROCÉDÉS DE PRÉPARATION D'ACIDE 5-BROMO-2-(3-CHLORO-PYRIDIN-2-YL)-2 H-PYRAZOLE-3-CARBOXYLIQUE

(30) Priority: 18.10.2019 US 201962916836 P; 06.11.2019 US 201962931320 P
(43) Date of publication of application: 24.08.2022
(73) Proprietor: FMC CORPORATION, Philadelphia, PA 19104 (US); FMC IP Technology GmbH, 8212 Neuhausen am Rheinfall (CH)
(72) Inventor: CAO, Yanchun, Philadelphia, PA 19104 (US); LIU, Xin, Philadelphia, PA 19104 (US); MAO, Jianhua, Philadelphia, PA 19104 (US); XU, Zhijian, Philadelphia, PA 19104 (US)
(74) Representative: Dehns
(86) International application number: PCT/US2020/055885
(87) International publication number: WO 2021/076832

(56) References cited:
- EP-A1- 2 145 885
- EP-A1- 2 189 455
- EP-A1- 2 719 697
- WO-A1-2004/067528
- WO-A1-2006/068669
- WO-A1-2012/030922
- WO-A1-2014/184343
- CN-A- 105 669 643
- US-A1- 2012 219 867
- US-A1- 2015 291 572
- FERNANDO BLANCO ET AL: "Substitution effects in N-pyrazole and N-imidazole derivatives along the periodic table", STRUCTURAL CHEMISTRY, KLUWER ACADEMIC PUBLISHERS-PLENUM PUBLISHERS, NE, vol. 18, no. 6, 26 September 2007 (2007-09-26), pages 965 - 975, XP019581080, ISSN: 1572-9001
- PIERCARLO FANTUCCI ET AL: "Electronic and geometrical structure of the pyrazole ligand co-ordinated to metal centres", JOURNAL OF THE CHEMICAL SOCIETY. DALTON TRANSACTIONS, no. 12, 1 January 1992 (1992-01-01), GB, pages 1981, XP055757251, ISSN: 0300-9246, DOI: 10.1039/dt9920001981
- R. MIETHCHEN R. RANDOW R. LISTEMANN J. HILDEBRANDT K. KOHLHEIM: "Micellaktivierte lodierung und partielle Dehalogenierung von Pyrarolen und 1,2,4-Triazolen Micelle Activated Reactions I. Micelle Activated Iodination and Partial Dehalogenation of Pyrazoles and 1,2,4-Triazoles", JOURNAL FÜR PRAKTISCHE CHEMIE, vol. 331, no. 5, 1 January 1989 (1989-01-01) - 1989, pages 799 - 805, XP055757253, Retrieved from the Internet <URL:https://doi.org/10.1002/prac.19893310514>

## Description

### FIELD OF INVENTION

This disclosure is directed to novel methods of synthesizing compounds of Formula VI, such as 5-Bromo-2-(3-chloro-pyridin-2-yl)-2H-pyrazole-3-carboxylic acid. Compounds prepared by the methods disclosed herein are useful for preparation of certain anthranilamide compounds that are of interest as insecticides, such as, for example, the insecticides chlorantraniliprole and cyantraniliprole.

### BACKGROUND

Conventional processes for the production of 5-Bromo-2-(3-chloro-pyridin-2-yl)-2H-pyrazole-3-carboxylic acid are subject to several industrial concerns, such as processability, environmental hazards, high cost, reagent reactivity, and necessary specialized equipment.

EP 2189455 A1 provides a method for producing a hydrazine compound which is useful as an insecticidal compound, production intermediates which can be suitably used for the production method, and methods for producing the intermediates. WO 2014/184343 A1 discloses a process for preparing N-substituted 1H-pyrazole-5-carboxylic acid compounds and derivatives thereof.

The present disclosure provides novel methods useful for preparing 5-Bromo-2-(3-chloro-pyridin-2-yl)-2H-pyrazole-3-carboxylic acid and derivatives thereof. The benefits of the methods of the present disclosure compared to previous methods are numerous and include improved overall yield, reduced cost, eliminated need for mixed solvent separations, reduced waste, simplified operation complexity, and reduced process hazards.

The disclosed methods provide an overall yield of about 50% with commercially available and easily handled reagents.

### BRIEF DESCRIPTION

The invention provides a method of preparing a compound of Formula VI as defined in the claims. In particular, provided herein is a method of preparing a compound of Formula VI 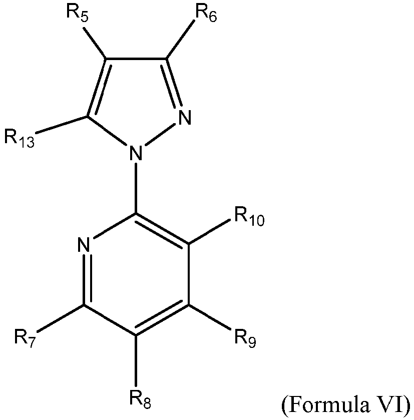
wherein each of R₅ - R₁₀ is independently selected from hydrogen and halogen; and
R₁₃ is an organic acid, the method comprising
   I) forming a mixture comprising
      A) a compound of Formula III 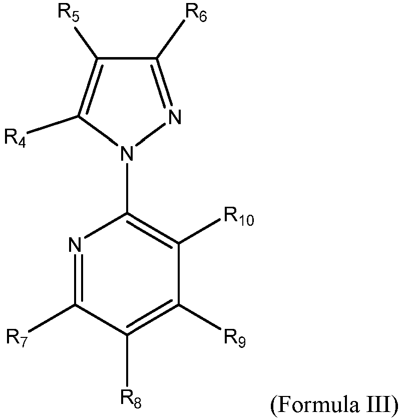
         wherein R₄ is hydrogen; and
         each of R₅ - R₁₀ is independently selected from hydrogen and halogen;
      B) a carbonyl-containing compound;
      C) a solvent;
      D) a compound comprising a metal; and
      E) optionally an additive; and
   II) reacting the mixture; and
      wherein the compound of Formula III is prepared by mixing a compound of Formula II with a compound of Formula IV in a solvent in the presence of an inorganic base and optionally an additive;
      wherein the compound of Formula II has the structure: 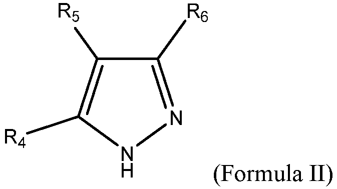
      wherein each of R₄, R₅, and R₆ is independently selected from hydrogen and halogen; and
      wherein at least one of R₄, R₅, and R₆ is hydrogen;
      wherein the compound of Formula IV has the structure: 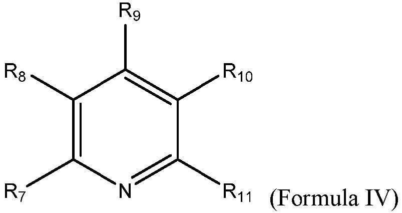
      wherein each of R₇ - R₁₁ is independently selected from hydrogen and halogen; and
      wherein the compound of Formula II is formed by
         i) forming a mixture comprising
            a) one or more compounds of Formula I 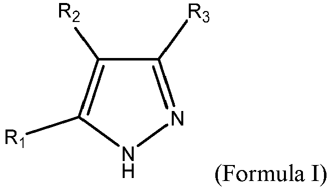
               wherein each of R₁, R₂, and R₃ is independently selected from halogen and hydrogen; and
               wherein at least two of R₁, R₂, and R₃ are halogen;
            b) optionally a dehalogenation reagent;
            c) a reducing agent; and
            d) a solvent; and
         ii) reacting the mixture.

The compound of Formula III, 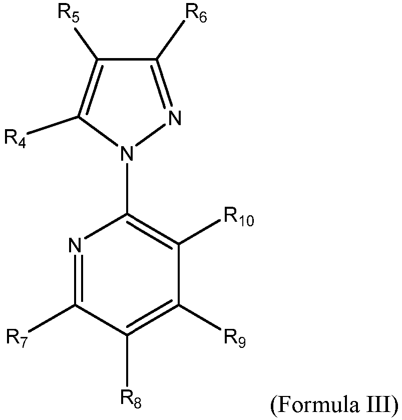
wherein R₄ is hydrogen; and
each of R₅ - R₁₀ is independently selected from hydrogen and halogen, may be prepared by a method comprising
   I) forming a mixture comprising
      A) a compound of Formula II, 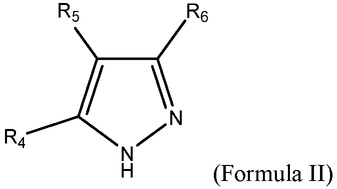
         wherein each of R₄, R₅, and R₆ is independently selected from hydrogen and halogen;
         wherein at least one of R₄, R₅, and R₆ is hydrogen; and
         wherein the compound of Formula II is prepared according to a method comprising
            i) forming a mixture comprising
               a) one or more compounds of Formula I, 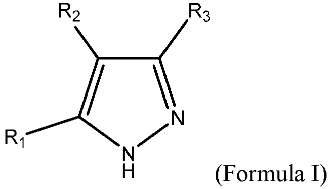
                  wherein each of R₁, R₂, and R₃ is independently selected from halogen and hydrogen; and
                  wherein at least two of R₁, R₂, and R₃ are halogen;
               b) optionally a dehalogenation reagent;
               c) a reducing agent; and
               d) a solvent; and
            ii) reacting the mixture.
      B) a compound of Formula IV, 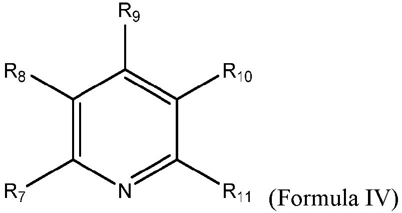 wherein each of R₇ - R₁₁ is independently selected from hydrogen and halogen;
      C) a solvent;
      D) an inorganic base; and
      E) optionally an additive; and
   II) reacting the mixture.

The compound of Formula II, 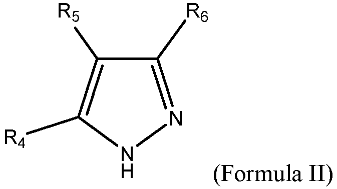
wherein each of R₄, R₅, and R₆ is independently selected from hydrogen and halogen; and
wherein at least one of R₄, R₅, and R₆ is hydrogen, may be prepared by a method comprising
   I) forming a mixture comprising
      A) one or more compounds of Formula I, wherein 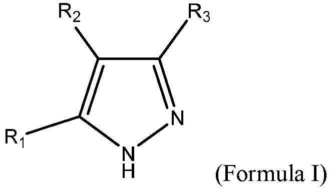
         each of R₁, R₂, and R₃ is independently selected from halogen and hydrogen;
         wherein at least two of R₁, R₂, and R₃ are halogen; and
         wherein the one or more compounds of Formula I are prepared according to a method comprising
            i) forming a mixture comprising
               a) pyrazole or a pyrazole derivative;
               b) a halogenation reagent;
               c) water; and
               d) optionally an inorganic base; and
            ii) reacting the mixture.
      B) optionally a dehalogenation reagent;
      C) a reducing agent; and
      D) a solvent; and
   II) reacting the mixture.

Compounds of Formula I, 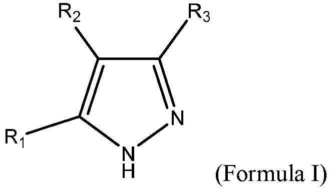
wherein each of R₁, R₂, and R₃ is independently selected from halogen and hydrogen; and
wherein at least two of R₁, R₂, and R₃ are halogen, may be prepared by a method comprising
   I) forming a mixture comprising
      A) pyrazole or a pyrazole derivative;
      B) a halogenation reagent;
      C) water; and
      D) optionally an inorganic base; and
   II) reacting the mixture.

Compounds of Formula II-A, 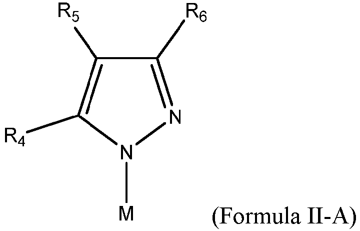
wherein M is selected from alkali metals and alkaline metals;
each of R₄, R₅, and R₆ is independently selected from hydrogen and halogen; and
wherein at least one of R₄, R₅, and R₆ is hydrogen, can be prepared by a method
comprising
   I) forming a mixture comprising
      A) one or more compounds of Formula I, wherein 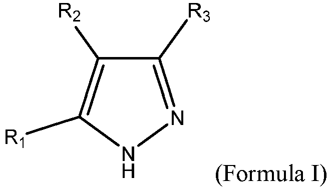
         each of R₁, R₂, and R₃ is independently selected from halogen and hydrogen;
         wherein at least two of R₁, R₂, and R₃ are halogen; and
         wherein the one or more compounds of Formula I are prepared according to a method comprising
            i) forming a mixture comprising
               a) pyrazole or a pyrazole derivative;
               b) a halogenation reagent;
               c) water; and
               d) optionally an inorganic base; and
            ii) reacting the mixture.
      B) optionally a dehalogenation reagent;
      C) a reducing agent; and
      D) a solvent; and
   II) reacting the mixture.

### DETAILED DESCRIPTION OF THE DISCLOSURE

As used herein, the terms "comprises," "comprising," "includes," "including," "has," "having," "contains", "containing," "characterized by" or any other variation thereof, are intended to cover a non-exclusive inclusion, subject to any limitation explicitly indicated. For example, a composition, mixture, process or method that comprises a list of elements is not necessarily limited to only those elements but may include other elements not expressly listed or inherent to such composition, mixture, process or method.

The transitional phrase "consisting of" excludes any element, step, or ingredient not specified. If in the claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole.

The transitional phrase "consisting essentially of" is used to define a composition or method that includes materials, steps, features, components, or elements, in addition to those literally disclosed, provided that these additional materials, steps, features, components, or elements do not materially affect the basic and novel characteristic(s) of the claimed invention. The term "consisting essentially of" occupies a middle ground between "comprising" and "consisting of".

Where an invention or a portion thereof is defined with an openended term such as "comprising," it should be readily understood that (unless otherwise stated) the description should be interpreted to also describe such an invention using the terms "consisting essentially of" or "consisting of."

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

As used herein, the term "about" means plus or minus 10% of the value.

The term "halogen", either alone or in compound words such as "haloalkyl", includes fluorine, chlorine, bromine or iodine. Further, when used in compound words such as "haloalkyl", said alkyl may be partially or fully substituted with halogen atoms which may be the same or different.

When a group contains a substituent which can be hydrogen, for example R⁴, then, when this substituent is taken as hydrogen, it is recognized that this is equivalent to said group being unsubstituted.

The term "organic base" includes, without limitation, amine compounds (e.g., primary, secondary and tertiary amines), heterocycles including nitrogen-containing heterocycles, and ammonium hydroxide.

The term "inorganic base" includes, without limitation, inorganic compounds with the ability to react with, or neutralize, acids to form salts, such as, for example, metal salts of hydroxide, carbonate, bicarbonate and phosphate.

The term "halogenation reagent" includes, without limitation, halogens and inorganic compounds, such as, for example, bromine, NBS, and 1,3-dibromo-5,5-dimethylhylhydantoin.

The term "phase transfer catalyst" includes compounds that facilitate the migration of a reactant from one phase into another phase where a reaction occurs. Phase transfer catalysis refers to the acceleration of the reaction upon the addition of the phase transfer catalyst.

The term "ester" includes, without limitation, a functional group comprising an ester bond (C(=O)-O-). In some aspects, the functional group comprising an ester bond is an alkyl (or cycloalkyl) having one to eight carbon atoms, like methyl, ethyl, 1 -propyl, 2-propyl, 1 - butyl, 1-methylheptyl (meptyl), etc.

The term "ether" includes, without limitation, a functional group comprising an ether bond (C-O-C).

The term "nitrile" includes, without limitation, a functional group comprising a nitrile bond (-C≡N).

The term "carboxylic acid" includes, without limitation, a functional group comprising a carboxylic acid bond (C(=O)-OH).

The term "organic acid" includes, without limitation, a functional group that confers acidity and consists of atoms selected from carbon, nitrogen, oxygen, and hydrogen.

Certain compounds formed in this invention can exist as one or more stereoisomers. The various stereoisomers include enantiomers, diastereomers, atropisomers and geometric isomers. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers.

The following embodiments 1-38 illustrate the invention and embodiments 39-128 are for reference only.

Embodiment 1. A method of preparing a compound of Formula VI, wherein 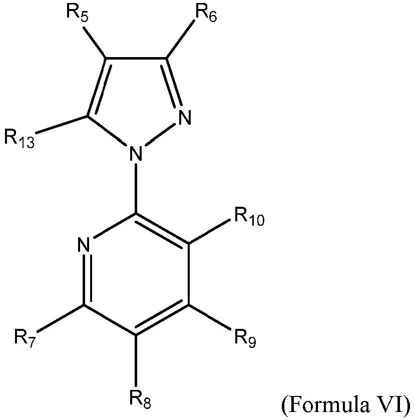
each of R₅ - R₁₀ is independently selected from hydrogen and halogen; and
R₁₃ is an organic acid, the method comprising
   I) forming a mixture comprising
      A) a compound of Formula III, wherein 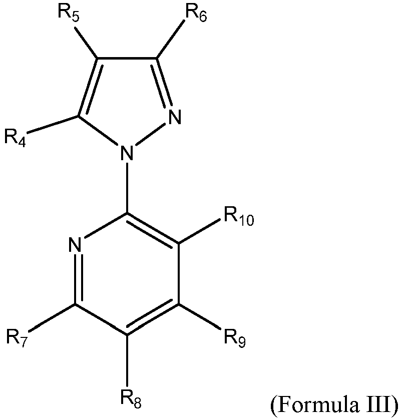
         R₄ is hydrogen; and
         each of R₅ - R₁₀ is independently selected from hydrogen and halogen;
      B) a carbonyl-containing compound;
      C) a solvent;
      D) a compound comprising a metal; and
      E) optionally an additive; and
   II) reacting the mixture; and
      wherein the compound of Formula III is prepared by mixing a compound of Formula II with a compound of Formula IV in a solvent in the presence of an inorganic base and optionally an additive;
      wherein the compound of Formula II has the structure: 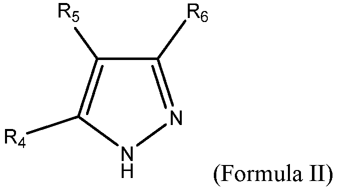
      wherein each of R₄, R₅, and R₆ is independently selected from hydrogen and halogen; and
      wherein at least one of R₄, R₅, and R₆ is hydrogen;
      wherein the compound of Formula IV has the structure: 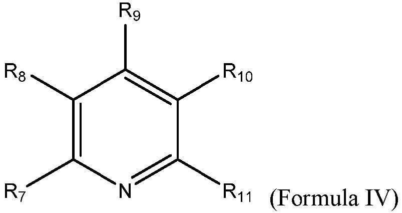
      wherein each of R₇ - R₁₁ is independently selected from hydrogen and halogen; and
      wherein the compound of Formula II is formed by
         i) forming a mixture comprising
            a) one or more compounds of Formula I 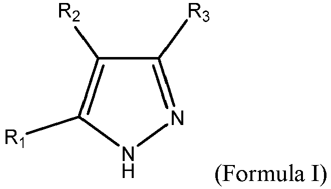
               wherein each of R₁, R₂, and R₃ is independently selected from halogen and hydrogen; and
               wherein at least two of R₁, R₂, and R₃ are halogen;
            b) optionally a dehalogenation reagent;
            c) a reducing agent; and
            d) a solvent; and
         ii) reacting the mixture.

Embodiment 2. The method of embodiment 1, wherein the compound comprising a metal is selected from a Grignard reagent and a lithium-containing compound.

Embodiment 3. The method of embodiment 2, wherein the Grignard reagent is selected from MeMgCl, iPrMgCl, iPrMgBr, EtMgCl, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof.

Embodiment 4. The method of embodiment 3, wherein the Grignard reagent is *i*Pr₂NMgCl.

Embodiment 5. The method of embodiment 2, wherein the lithium-containing compound is selected from LDA, nBuLi, and combinations thereof.

Embodiment 6. The method of embodiment 1, wherein the solvent is selected from THF, toluene, 1,4-dioxane, Me-THF, and combinations thereof.

Embodiment 7. The method of embodiment 6, wherein the solvent is THF.

Embodiment 8. The method of embodiment 1, wherein the carbonyl-containing compound is selected from dimethylcarbonate, N,N-dimethylacetamide, carbon dioxide, and combinations thereof.

Embodiment 9. The method of embodiment 8, wherein the carbonyl-containing compound is carbon dioxide.

Embodiment 10. The method of embodiment 1, wherein the method step of reacting the mixture occurs at a reaction temperature in the range of about 0 °C to about 60°C.

Embodiment 11. The method of embodiment 10, wherein the method step of reacting the mixture occurs at a reaction temperature in the range of about 0 °C to about 30°C.

Embodiment 12. The method of embodiment 1, wherein R₅ and R₆ of Formula III are each independently hydrogen.

Embodiment 13. The method of embodiment 1, wherein the compound of Formula III, is prepared according to a method comprising
I) forming a mixture comprising
   A) a compound of Formula II, wherein 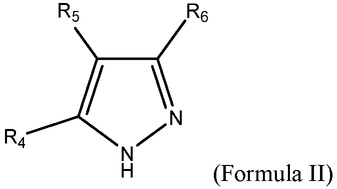
      each of R₄, R₅, and R₆ is independently selected from hydrogen and halogen; and
      wherein at least one of R₄, R₅, and R₆ is hydrogen;
   B) a compound of Formula IV, wherein 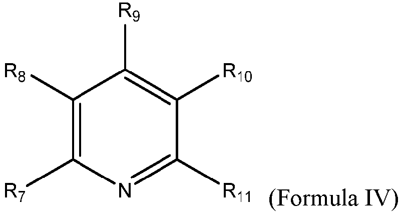 each of R₇ - R₁₁ is independently selected from hydrogen and halogen;
   C) a solvent;
   D) an inorganic base; and
   E) optionally an additive; and
II) reacting the mixture.

Embodiment 14. The method of embodiment 13, wherein the inorganic base is selected from powder sodium hydroxide, powder potassium hydroxide, potassium carbonate, potassium phosphate, powder sodium methoxide, powder potassium t-butoxide, and combinations thereof.

Embodiment 15. The method of embodiment 13, wherein the solvent is selected from toluene, N,N-dimethylformamide, N,N-dimethylacetamide, N-methyl-2-pyrrolidone, acetonitrile, and combinations thereof.

Embodiment 16. The method of embodiment 13, wherein the additive is selected from potassium iodide, a phase transfer catalyst, and combinations thereof.

Embodiment 17. The method of embodiment 16, wherein the phase transfer catalyst is selected from butyl ammonium chloride, tetra butyl ammonium bromide, aliquat-336, 18-crown-6, and combinations thereof.

Embodiment 18. The method of embodiment 13, wherein the method step of reacting the mixture occurs at a reaction temperature in the range of about 100 °C to about 200 °C.

Embodiment 20. The method of embodiment **1,** wherein the solvent is selected from acetic acid, water, toluene, N,N-dimethylformamide, N,N-dimethylacetamide, and combinations thereof.

Embodiment 21. The method of embodiment **1,** wherein the reducing agent is selected from sodium sulfite, sodium bisulfite, sodium hyposulfite, sodium thiosulfate, sodium hydrosulfide, sodium sulfate, and combinations thereof.

Embodiment 22. The method of embodiment 1, wherein the dehalogenation reagent is selected from sodium iodide, iodine, potassium iodide, tetra-n-butyl ammonium iodide, and combinations thereof.

Embodiment 23. The method of embodiment **1,** wherein the method step of reacting the mixture occurs at a reaction temperature in the range of about 100 °C to about 180 °C.

Embodiment 24. The method of embodiment **1,** wherein the one or more compounds of Formula I are prepared according to a method comprising
I) forming a mixture comprising
   A) pyrazole or a pyrazole derivative;
   B) a halogenation reagent;
   C) water; and
   D) optionally an inorganic base; and
II) reacting the mixture.

Embodiment 25. The method of embodiment 24, wherein the halogenation reagent comprises
A) a reagent selected from hydrogen bromide, bromine, N-bromosuccinimide, 1,3-dibromo-5,5-dimethylhylhydantoin, sodium bromide, potassium bromide, and combinations thereof; and
B) optionally hydrogen peroxide.

Embodiment 26. The method of embodiment 24, wherein the inorganic base is selected from powder sodium hydroxide, sodium hydroxide solution, powder sodium acetate, and combinations thereof.

Embodiment 27. The method of embodiment 24, wherein the method step of reacting the mixture occurs at a reaction temperature in the range of about 0 °C to about 70 °C.

Embodiment 28. The method of embodiment 24, wherein the halogenation reagent is a mixture of about 1 part by weight hydrogen peroxide and about 2 parts by weight hydrogen bromide.

The compound of Formula III may be prepared by a method as defined in Embodiments 29-43 below.

Embodiment 29. A method of preparing a compound of Formula III, wherein 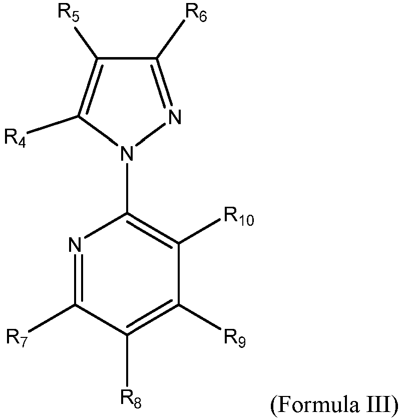
R₄ is hydrogen; and
each of R₅ - R₁₀ is independently selected from hydrogen and halogen, the method comprising
   I) forming a mixture comprising
      A) a compound of Formula II, wherein 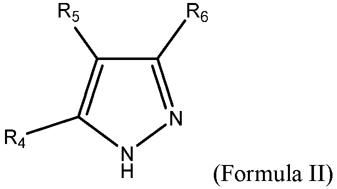
         each of R₄, R₅, and R₆ is independently selected from hydrogen and halogen;
         wherein at least one of R₄, R₅, and R₆ is hydrogen; and
         wherein the compound of Formula II is prepared according to a method comprising
            i) forming a mixture comprising
               a) one or more compounds of Formula I, wherein 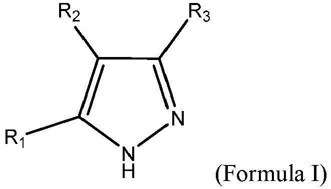
                  each of R₁, R₂, and R₃ is independently selected from halogen and hydrogen; and
                  wherein at least two of R₁, R₂, and R₃ are halogen;
               b) optionally a dehalogenation reagent;
               c) a reducing agent; and
               d) a solvent; and
            ii) reacting the mixture.
      B) a compound of Formula IV, wherein 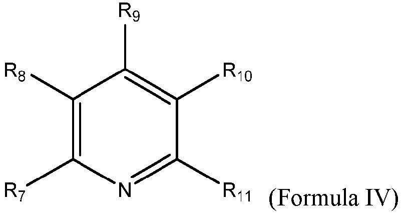 each of R₇ - R₁₁ is independently selected from hydrogen and halogen;
      C) a solvent;
      D) an inorganic base; and
      E) optionally an additive; and
   II) reacting the mixture.

Embodiment 30. The method of embodiment 29, wherein the inorganic base is selected from powder sodium hydroxide, powder potassium hydroxide, potassium carbonate, potassium phosphate, powder sodium methoxide, powder potassium t-butoxide, and combinations thereof.

Embodiment 31. The method of embodiment 29, wherein the solvent C) is selected from toluene, N,N-dimethylformamide, N,N-dimethylacetamide, N-methyl-2-pyrrolidone, acetonitrile, and combinations thereof.

Embodiment 32. The method of embodiment 29, wherein the additive is selected from potassium iodide, a phase transfer catalyst, and combinations thereof.

Embodiment 33. The method of embodiment 32, wherein the phase transfer catalyst is selected from butyl ammonium chloride, tetra butyl ammonium bromide, aliquat-336, 18-crown-6, and combinations thereof.

Embodiment 34. The method of embodiment 29, wherein the method step II) of reacting the mixture occurs at a reaction temperature in the range of about 100 °C to about 200 °C.

Embodiment 35. The method of embodiment 29, wherein the solvent d) is selected from acetic acid, water, toluene, N,N-dimethylformamide, N,N-dimethylacetamide, and combinations thereof.

Embodiment 36. The method of embodiment 29, wherein the reducing agent is selected from sodium sulfite, sodium bisulfite, sodium hyposulfite, sodium thiosulfate, sodium hydrosulfide, sodium sulfate, and combinations thereof.

Embodiment 37. The method of embodiment 29, wherein the dehalogenation reagent is selected from sodium iodide, iodine, potassium iodide, tetra-n-butyl ammonium iodide, and combinations thereof.

Embodiment 38. The method of embodiment 29, wherein the method step ii) of reacting the mixture occurs at a reaction temperature in the range of about 100 °C to about 180 °C.

Embodiment 39. The method of embodiment 29, wherein the one or more compounds of Formula I are prepared according to a method comprising
I) forming a mixture comprising
   A) pyrazole or a pyrazole derivative;
   B) a halogenation reagent;
   C) water; and
   D) optionally an inorganic base; and
II) reacting the mixture.

Embodiment 40. The method of embodiment 39, wherein the halogenation reagent comprises
A) a reagent selected from hydrogen bromide, bromine, N-bromosuccinimide, 1,3-dibromo-5,5-dimethylhylhydantoin, sodium bromide, potassium bromide, and combinations thereof; and
B) optionally hydrogen peroxide.

Embodiment 41. The method of embodiment 39, wherein the inorganic base is selected from powder sodium hydroxide, sodium hydroxide solution, powder sodium acetate, and combinations thereof.

Embodiment 42. The method of embodiment 39, wherein the method step of reacting the mixture occurs at a reaction temperature in the range of about 0 °C to about 70 °C.

Embodiment 43. The method of embodiment 39, wherein the halogenation reagent is a mixture of about 1 part by weight hydrogen peroxide and about 2 parts by weight hydrogen bromide.

The compound of Formula II may be prepared by a method as defined in Embodiments 44-52 below.

Embodiment 44. A method of preparing a compound of Formula II, wherein 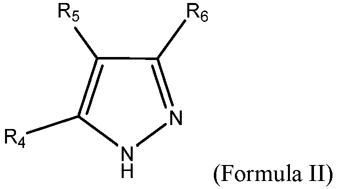
each of R₄, R₅, and R₆ is independently selected from hydrogen and halogen; and
wherein at least one of R₄, R₅, and R₆ is hydrogen, the method comprising
   I) forming a mixture comprising
      A) one or more compounds of Formula I, wherein 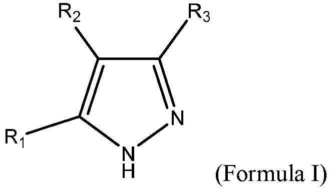
         each of R₁, R₂, and R₃ is independently selected from halogen and hydrogen;
         wherein at least two of R₁, R₂, and R₃ are halogen; and
         wherein the one or more compounds of Formula I are prepared according to a method comprising
            i) forming a mixture comprising
               a) pyrazole or a pyrazole derivative;
               b) a halogenation reagent;
               c) water; and
               d) optionally an inorganic base; and
            ii) reacting the mixture.
      B) optionally a dehalogenation reagent;
      C) a reducing agent; and
      D) a solvent; and
   II) reacting the mixture.

Embodiment 45. The method of embodiment 44, wherein the solvent is selected from acetic acid, water, toluene, N,N-dimethylformamide, N,N-dimethylacetamide, and combinations thereof.

Embodiment 46. The method of embodiment 44, wherein the reducing agent is selected from sodium sulfite, sodium bisulfite, sodium hyposulfite, sodium thiosulfate, sodium hydrosulfide, sodium sulfate, and combinations thereof.

Embodiment 47. The method of embodiment 44, wherein the dehalogenation reagent is selected from sodium iodide, iodine, potassium iodide, tetra-n-butyl ammonium iodide, and combinations thereof.

Embodiment 48. The method of embodiment 44, wherein the method step II) of reacting the mixture occurs at a reaction temperature in the range of about 100 °C to about 180 °C.

Embodiment 49. The method of embodiment 44, wherein the halogenation reagent comprises
A) a reagent selected from hydrogen bromide, bromine, N-bromosuccinimide, 1,3-dibromo-5,5-dimethylhylhydantoin, sodium bromide, potassium bromide, and combinations thereof; and
B) optionally hydrogen peroxide.

Embodiment 50. The method of embodiment 44, wherein the inorganic base is selected from powder sodium hydroxide, sodium hydroxide solution, powder sodium acetate, and combinations thereof.

Embodiment 51. The method of embodiment 44, wherein the method step ii) of reacting the mixture occurs at a reaction temperature in the range of about 0 °C to about 70 °C.

Embodiment 52. The method of embodiment 44, wherein the halogenation reagent is a mixture of about 1 part by weight hydrogen peroxide and about 2 parts by weight hydrogen bromide.

The compound of Formula I may be prepared by a method as defined in Embodiments 53-57 below.

Embodiment 53. A method of preparing one or more compounds of Formula I, wherein 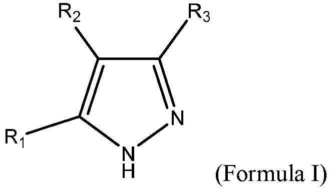
each of R₁, R₂, and R₃ is independently selected from halogen and hydrogen; and
wherein at least two of R₁, R₂, and R₃ are halogen, the method comprising
   I) forming a mixture comprising
      A) pyrazole or a pyrazole derivative;
      B) a halogenation reagent;
      C) water; and
      D) optionally an inorganic base; and
   II) reacting the mixture.

Embodiment 54. The method of embodiment 53, wherein the halogenation reagent comprises
A) a reagent selected from hydrogen bromide, bromine, N-bromosuccinimide, 1,3-dibromo-5,5-dimethylhylhydantoin, sodium bromide, potassium bromide, and combinations thereof; and
B) optionally hydrogen peroxide.

Embodiment 55. The method of embodiment 53, wherein the inorganic base is selected from powder sodium hydroxide, sodium hydroxide solution, powder sodium acetate, and combinations thereof.

Embodiment 56. The method of embodiment 53, wherein the method step of reacting the mixture occurs at a reaction temperature in the range of about 0 °C to about 70 °C.

Embodiment 57. The method of embodiment 53, wherein the halogenation reagent is a mixture of about 1 part by weight hydrogen peroxide and about 2 parts by weight hydrogen bromide.

Compounds of Formula II-A can be prepared by a method as defined in Embodiments 118-128 below.

Embodiment 118. A method of preparing a compound of Formula II-A, wherein 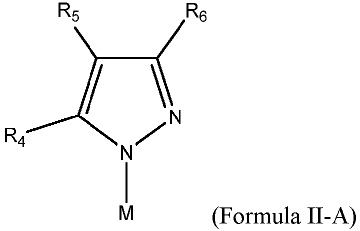
M is selected from alkali metals and alkaline metals;
each of R₄, R₅, and R₆ is independently selected from hydrogen and halogen; and
wherein at least one of R₄, R₅, and R₆ is hydrogen, the method comprising
   I) forming a mixture comprising
      A) one or more compounds of Formula I, wherein 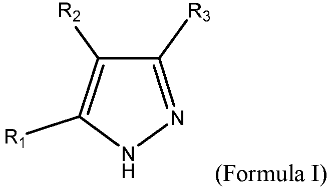
         each of R₁, R₂, and R₃ is independently selected from halogen and hydrogen;
         wherein at least two of R₁, R₂, and R₃ are halogen; and
         wherein the one or more compounds of Formula I are prepared according to a method comprising
            i) forming a mixture comprising
               a) pyrazole or a pyrazole derivative;
               b) a halogenation reagent;
               c) water; and
               d) optionally an inorganic base; and
            ii) reacting the mixture.
      B) optionally a dehalogenation reagent;
      C) a reducing agent; and
      D) a solvent; and
   II) reacting the mixture.

Embodiment 119. The method of embodiment 118, wherein the solvent is selected from acetic acid, water, toluene, N,N-dimethylformamide, N,N-dimethylacetamide, and combinations thereof.

Embodiment 120. The method of embodiment 118, wherein the reducing agent is selected from sodium sulfite, sodium bisulfite, sodium hyposulfite, sodium thiosulfate, sodium hydrosulfide, sodium sulfate, and combinations thereof.

Embodiment 121. The method of embodiment 118, wherein the dehalogenation reagent is selected from sodium iodide, iodine, potassium iodide, tetra-n-butyl ammonium iodide, and combinations thereof.

Embodiment 122. The method of embodiment 118, wherein the method step II) of reacting the mixture occurs at a reaction temperature in the range of about 100 °C to about 180 °C.

Embodiment 123. The method of embodiment 118, wherein the halogenation reagent comprises
A) a reagent selected from hydrogen bromide, bromine, N-bromosuccinimide, 1,3-dibromo-5,5-dimethylhylhydantoin, sodium bromide, potassium bromide, and combinations thereof; and
B) optionally hydrogen peroxide.

Embodiment 124. The method of embodiment 118, wherein the inorganic base is selected from powder sodium hydroxide, sodium hydroxide solution, powder sodium acetate, and combinations thereof.

Embodiment 125. The method of embodiment 118, wherein the method step ii) of reacting the mixture occurs at a reaction temperature in the range of about 0 °C to about 70 °C.

Embodiment 126. The method of embodiment 118, wherein the halogenation reagent is a mixture of about 1 part by weight hydrogen peroxide and about 2 parts by weight hydrogen bromide.

Embodiment 127. The method of embodiment 118, wherein M is selected from lithium, sodium, potassium, calcium, and magnesium.

Embodiment 128. The method of embodiment 118, wherein the compound of Formula II-A is

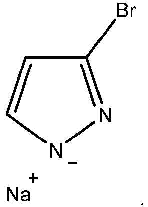

In one aspect, a compound of Formula VI is prepared according to a method represented by Scheme 3. The R groups are as defined anywhere in this disclosure.

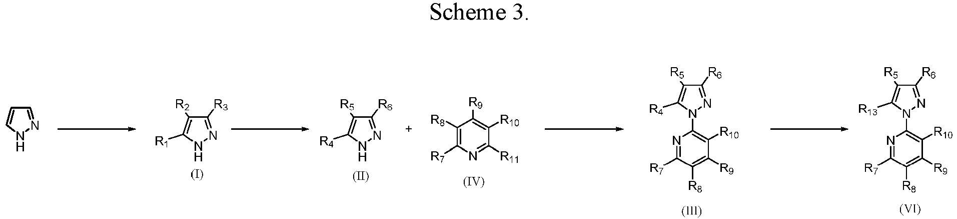

In one aspect, a compound of Formula VI is prepared according to a method represented by Scheme 4. The R groups are as defined anywhere in this disclosure. Formula IA and Formula IB represent two distinct compounds of Formula I.

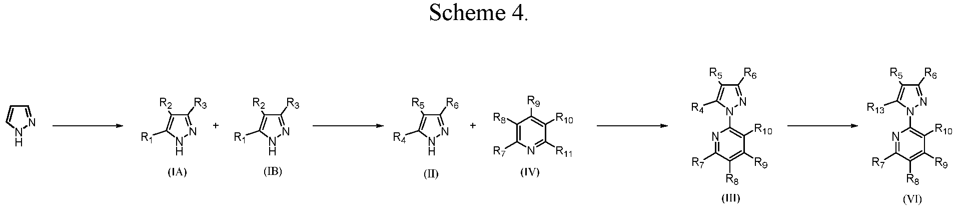

In one aspect, 5-Bromo-2-(3-chloro-pyridin-2-yl)-2H-pyrazole-3-carboxylic acid is prepared according to a method represented by Scheme 5.

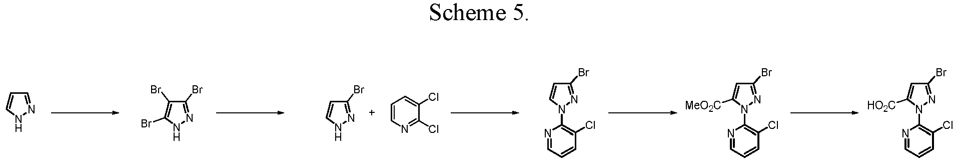

In one aspect, 5-Bromo-2-(3-chloro-pyridin-2-yl)-2H-pyrazole-3-carboxylic acid is prepared according to a method represented by Scheme 6.

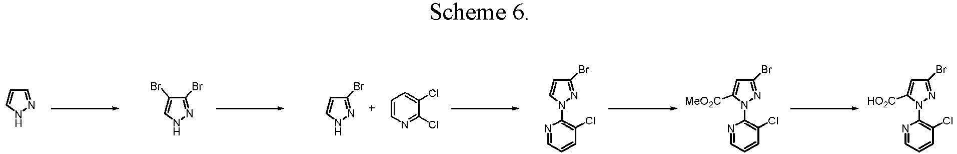

In one aspect, 5-Bromo-2-(3-chloro-pyridin-2-yl)-2H-pyrazole-3-carboxylic acid is prepared according to a method represented by Scheme 7.

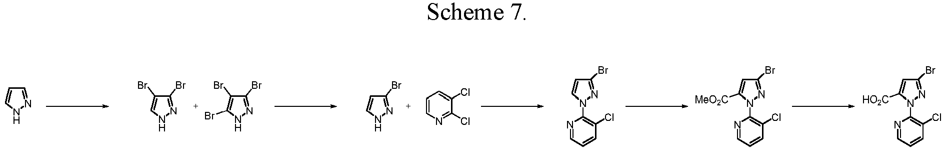

In one aspect, 5-Bromo-2-(3-chloro-pyridin-2-yl)-2H-pyrazole-3-carboxylic acid is prepared according to a method represented by Scheme 8.

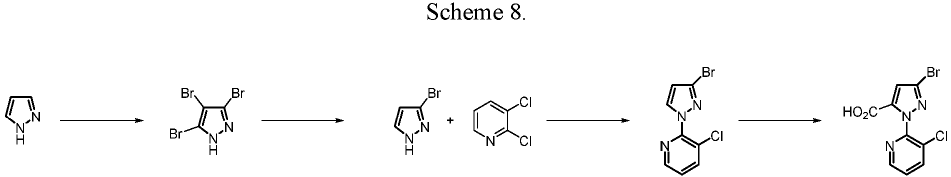

In one aspect, 5-Bromo-2-(3-chloro-pyridin-2-yl)-2H-pyrazole-3-carboxylic acid is prepared according to a method represented by Scheme 9.

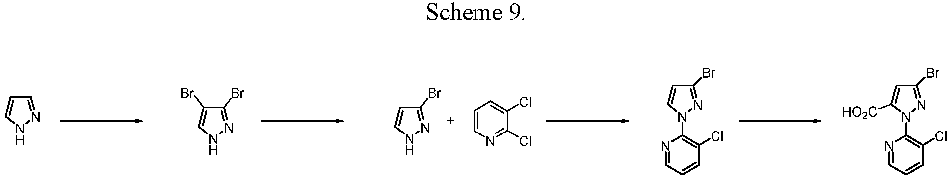

In one aspect, 5-Bromo-2-(3-chloro-pyridin-2-yl)-2H-pyrazole-3-carboxylic acid is prepared according to a method represented by Scheme 10.

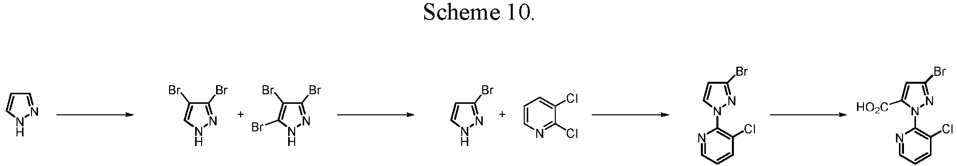

At least one distinct compound of Formula I may be prepared according to a method represented by Scheme 11. The R groups are as defined anywhere in this disclosure.

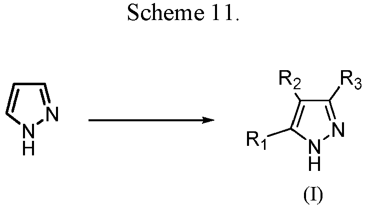

This method includes reacting pyrazole with a halogenation reagent in water and optionally in the presence of an inorganic base. In one embodiment, the halogenation reagent is selected from hydrogen peroxide/HBr, bromine (Br₂), N-bromosuccinimide (NBS), 1,3-dibromo-5,5-dimethylhylhydantoin, hydrogen peroxide/NaBr, hydrogen peroxide/KBr, hydrogen peroxide/Br₂, and combinations thereof. In another embodiment, the halogenation reagent is hydrogen peroxide/HBr. In one embodiment, the inorganic base is selected from powder sodium hydroxide, sodium hydroxide solution, powder sodium acetate, and combinations thereof. In another embodiment, there is no base. In one embodiment, the reaction temperature is in the range from about 0 °C to about 40 °C. In another embodiment, the reaction temperature is in the range from about 0 °C to 20 °C. The relative amounts of distinct compounds of Formula I produced by the reaction can be controlled according to the ratio of hydrogen peroxide/HBr. In one embodiment, hydrogen bromide and hydrogen peroxide and pyrazole are present in a ratio in the range from about 2 eq: 2 eq: 1 eq to about 10 eq: 2 eq: 1 eq. In another embodiment, hydrogen bromide and hydrogen peroxide and pyrazole are present in a ratio of 4 eq: 2 eq: 1 eq. In one embodiment, the halogenation reagent is a mixture of about 1 part by weight hydrogen peroxide and about 1 part by weight hydrogen bromide. In another embodiment, the halogenation reagent is a mixture of about 1 part by weight hydrogen peroxide and about 2 parts by weight hydrogen bromide. In another embodiment, the halogenation reagent is a mixture of about 1 part by weight hydrogen peroxide and about 5 parts by weight hydrogen bromide.

A compound of Formula II may be prepared according to a method represented by Scheme 12. The R groups are as defined anywhere in this disclosure.

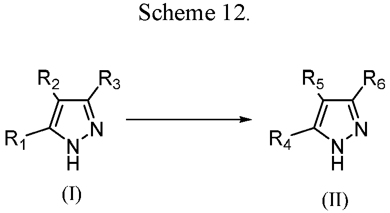

This method includes reacting at least one distinct compound of Formula I with a dehalogenation reagent in a solvent in the presence of a reducing agent. In one embodiment, the solvent is selected from water, acetic acid, toluene, N,N-Dimethylformamide (DMF), N,N-Dimethylacetamide(DMAc), and combinations thereof. In a preferred embodiment, the solvent is water. In another embodiment, the dehalogenation reagent is selected from sodium iodide, iodine, potassium iodide, tetra-n-butyl ammonium iodide (TBAI), and combinations thereof. In another embodiment, the dehalogenation reagent is potassium iodide. In one embodiment, the reducing agent is selected from sodium sulfite, sodium bisulfite, sodium hyposulfite, sodium thiosulfate, sodium hydrosulfide, sodium sulphate, and combinations thereof. In another embodiment, the reducing agent is sodium sulfite. In one embodiment, the reaction temperature is in the range from about 100 °C to about 180 °C. In another preferred embodiment, the reaction temperature ranges from about 160 °C to about 180 °C.

A compound of Formula II-A may be prepared according to a method represented by Scheme 13. The R groups are as defined anywhere in this disclosure.

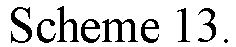

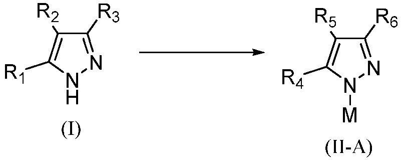

The compound of Formula II-A may be a metal salt of Formula II. The bond between M and N may be an ionic bond. M may be selected from alkali metals and alkaline metals. M may be selected from lithium, sodium, and potassium, such as sodium. Alternatively, M may be selected from calcium and magnesium.

For example, the compound of Formula II-A can be

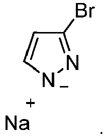

This method includes reacting at least one distinct compound of Formula I with a dehalogenation reagent in a solvent in the presence of a reducing agent. The solvent may be selected from water, acetic acid, toluene, N,N-Dimethylformamide (DMF), N,N-Dimethylacetamide(DMAc), and combinations thereof, such as water. The dehalogenation reagent may be selected from sodium iodide, iodine, potassium iodide, tetra-n-butyl ammonium iodide (TBAI), and combinations thereof. The dehalogenation reagent may be potassium iodide. The reducing agent may be selected from sodium sulfite, sodium bisulfite, sodium hyposulfite, sodium thiosulfate, sodium hydrosulfide, sodium sulphate, and combinations thereof, such as sodium sulfite. The reaction temperature may be in the range from about 100 °C to about 180 °C, such as the reaction temperature ranging from about 160 °C to about 180 °C.

According to the invention, a compound of Formula III is prepared according to a method represented by Scheme 14. The R groups are as defined anywhere in this disclosure.

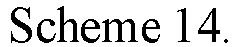

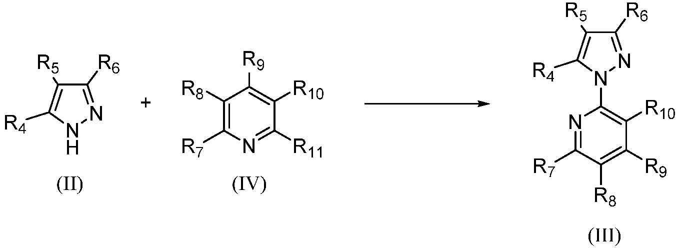

This method includes mixing a compound of Formula II with a compound of Formula IV in a solvent in the presence of an inorganic base and optionally an additive. In one embodiment, the inorganic base is selected from powder sodium hydroxide, powder potassium hydroxide, potassium carbonate, potassium phosphate, powder sodium methoxide, powder potassium t-butoxide, and combinations thereof. In one embodiment, the solvent is selected from toluene, N,N-Dimethylformamide (DMF), N,N-Dimethylacetamide (DMAc) and N-methyl-2-pyrrolidone (NMP), acetonitrile, and combinations thereof. In another embodiment, the solvent is toluene. In one embodiment, the reaction temperature is in the range from about 100 °C to about 200 °C. In another embodiment, the reaction temperature is in the range from about 130 °C to about 180 °C. In another embodiment, the temperature is 145 °C to about 160 °C.

In one aspect, a compound of Formula VI is prepared according to a method represented by Scheme 15. The R groups are as defined anywhere in this disclosure.

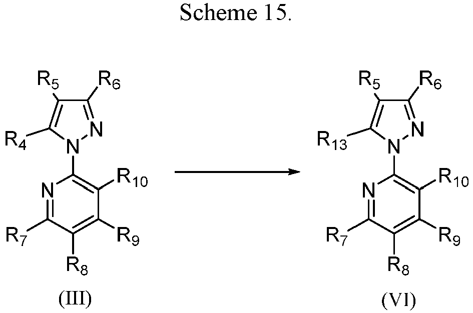

This aspect include mixing a compound of Formula III with CO₂ in a solvent in the presence of a base reagent and optionally an additive. In one embodiment, the base reagent is selected from MeMgCl, iPrMgCl, *i*PrMgBr, EtMgCl, LDA, nBuLi, iPr₂NMgCl, *i*Pr₂NMgBr, Et₂NMgCl, TMPMgCl, TMPMgCl•LiCl, iPr₂NMgCl•LiCl, *i*Pr₂NMgBr•LiCl, and combinations thereof. In another embodiment, the base reagent is *i*Pr₂NMgCl. In one embodiment, the solvent is selected from THF, toluene, 1,4-dioxane, Me-THF, and combinations thereof. In another embodiment, the solvent is THF. In one embodiment, the reaction temperature is in the range from about 0 °C to about 60 °C. In another embodiment, the temperature is in the range from about 0 °C to about 30 °C.

### EXAMPLES

Without further elaboration, it is believed that one skilled in the art using the preceding description can utilize the present invention to its fullest extent. The following Examples are, therefore, to be construed as merely illustrative, and not limiting of the disclosure in any way whatsoever. The starting material for the following Examples may not have necessarily been prepared by a particular preparative run whose procedure is described in other Examples. It also is understood that any numerical range recited herein includes all values from the lower value to the upper value. For example, if a range is stated as 10-50, it is intended that values such as 12-30, 20-40, or 30-50, etc., are expressly enumerated in this specification. These are only examples of what is specifically intended, and all possible combinations of numerical values between and including the lowest value and the highest value enumerated are to be considered to be expressly stated in this application.

### Reference Example 1. Hydrogen peroxide/HBr as a halogenation reagent.

34 grams of pyrazole was dissolved in 100 g water and charged to a reactor with 337.2 g of 48% hydrogen bromide solution. 170 g of 30% hydrogen peroxide was added drop-wise at 0 °C over 2 hours. The reaction temperature was controlled at 0-20 °C. After reaction, the product was precipitated as a solid, and then the reaction mixture was quenched with 10% sodium sulfite. After filtration and drying, 142 g of high purity (97%, LC Area) of a mixture of 3,4,-dibromo-1H-pyrazole and 3,4,5-tribromo-1H-pyrazole is obtained (the ratio of 3,4-dibromo-1H-pyrazole to 3,4,5-tribromo-1H-pyrazole = 9:1, LC Area).

### Reference

### Example 2. Bromine/sodium hydroxide as a halogenation reagent.

34 grams of pyrazole was dissolved in water and then sodium hydroxide was added at 0 °C to obtain the corresponding pyrazole sodium salt. Next, 239.7 g of bromine was added drop-wise at 0 °C over 2 hours. The reaction temperature was controlled at 20-40 °C. After reaction, the product was precipitated as a solid, and then the reaction mixture was quenched with 10% sodium sulfite. After filtration and drying, 147 g of high purity (98%, LC Area) of 3,4,5-tribromo-1H-pyrazole was obtained.

### Example 3. Potassium iodide/sodium sulfite as a dehalogenation reagent.

100 grams of a mixture of 3,4,5-tribromo-1H-pyrazole and 3,4,-dibromo-1H-pyrazole (the ratio of 3,4-dibromo-1H-pyrazole to 3,4,5-tribromo-1H-pyrazole = 9:1, LC Area), 111g of Na₂SO₃ in 500 mL water were reacted at 160-180 °C for 4 hours to complete reaction. After completion of the reaction, the reaction mixture was extracted with methyl isobutyl ketone (MIBK), and concentrated under vacuum at 40-45 °C to obtain 3-bromo-1H-pyrazole as a solid.

### Example 4. Coupling reaction.

11.5 grams of 3-bromo-1H-pyrazole and 14.7 g of 40% NaOH solution were reacted in the presence of toluene at a temperature of 150-160 °C. Water was removed by azetropic distillation under reflux temperature to yield the corresponding 3-bromo-1H-pyrazole sodium salt. Then, toluene and 13.5 g of 2,3-dichloropyridine were added and the mixture was reacted at 150-160 °C. After reaction, the reaction mixture was quenched with water and the organic layer was separated. The organic layer was then washed with water and concentrated under vacuum at 40-45 °C to obtain 2-(3-bromo-1H-pyrazol-1-yl)-3-chloropyridine as a solid.

### Example 5. Reaction in the presence of a Grignard reagent.

32 grams of 2-(3-bromo-1H-pyrazol-1-yl)-3-chloropyridine was dissolved in THF then iPr₂NMgCl (in situ MeMgCl and iPr₂NH) was added at 0 °C to yield the corresponding 2-(3-bromo-1H-pyrazol-1-yl)-3-chloropyridine magnesium salt. After 2.5 hours, 52.0 g of DMC was added drop-wise at room temperature over 6 hours. The reaction temperature was controlled at 20-40 °C. After reaction, THF and DMC were distilled off under reduced pressure, and then the reaction mixture was quenched with water. Next, toluene was added. After separation and concentration, 36.7 g of high purity (90%, LC Area) of methyl 3-bromo-1-(3-chloropyridin-2-yl)-1H-pyrazole-5-carboxylate was obtained.

### Example 6. Hydrolysis.

30 grams of methyl 3-bromo-1-(3-chloropyridin-2-yl)-1H-pyrazole-5-carboxylate and a caustic soda solution in 90 g of toluene were charged to a flask at 85°C. The the mixture was kept at 80-85 °C for 2 hours to complete reaction. The aqueous phase was washed with toluene twice. Dilute H₂SO₄ was used to adjust pH to about 1. After filtration and drying, 26.5 g (97%, LC Area) of 5-bromo-2-(3-chloro-pyridin-2-yl)-2H-pyrazole-3-carboxylic acid was obtained.

### Example 7. Carboxylation.

32 grams of 2-(3-bromo-1H-pyrazol-1-yl)-3-chloropyridine was dissolved in THF and then iPr₂NMgCl, obtained in situ by separately adding MeMgCl and iPr₂NH, was added at 0 °C to yield the corresponding 2-(3-bromo-1H-pyrazol-1-yl)-3-chloropyridine magnesium salt. After 2.5 hours, CO₂ gas was added at room temperature over 1 hour. The reaction temperature was controlled at 20-40 °C. After reaction, THF was distilled off under reduced pressure, and then the reaction mixture was quenched with water. Next, toluene was added. After separation and concentration, 30.0 g of high purity (95%, LC Area) of 5-bromo-2-(3-chloro-pyridin-2-yl)-2H-pyrazole-3-carboxylic acid was obtained.

This written description uses examples to illustrate the present disclosure, including the best mode, and also to enable any person skilled in the art to practice the disclosure, including making and using any devices or systems and performing any incorporated methods. The scope of the invention is defined by the following claims.

## Claims

1. A method of preparing a compound of Formula VI 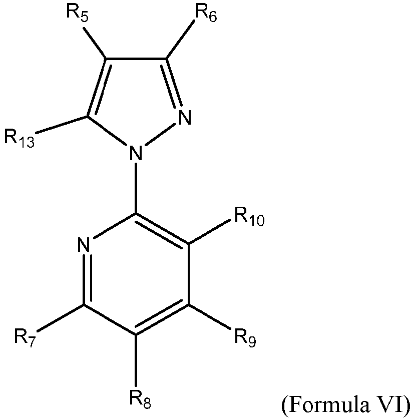
wherein each of R₅ - R₁₀ is independently selected from hydrogen and halogen; and
R₁₃ is an organic acid, the method comprising
I) forming a mixture comprising
A) a compound of Formula III 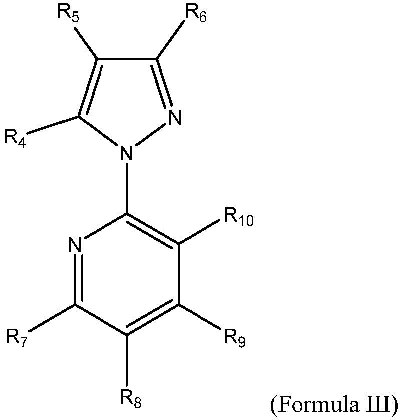
wherein R₄ is hydrogen; and
each of R₅ - R₁₀ is independently selected from hydrogen and halogen;
B) a carbonyl-containing compound;
C) a solvent;
D) a compound comprising a metal; and
E) optionally an additive; and
II) reacting the mixture; and
wherein the compound of Formula III is prepared by mixing a compound of Formula II with a compound of Formula IV in a solvent in the presence of an inorganic base and optionally an additive;
wherein the compound of Formula II has the structure: 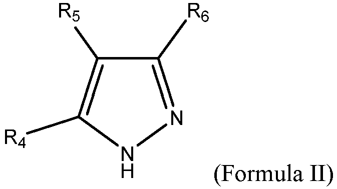
wherein each of R₄, R₅, and R₆ is independently selected from hydrogen and halogen; and
wherein at least one of R₄, R₅, and R₆ is hydrogen;
wherein the compound of Formula IV has the structure: 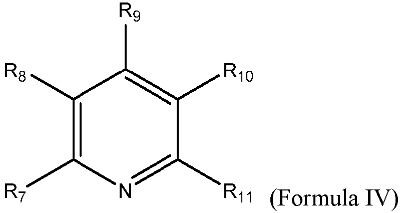
wherein each of R₇ - R₁₁ is independently selected from hydrogen and halogen; and
wherein the compound of Formula II is formed by
i) forming a mixture comprising
a) one or more compounds of Formula I 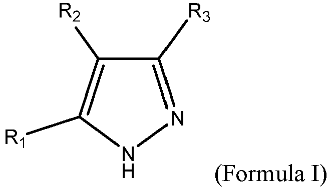
wherein each of R₁, R₂, and R₃ is independently selected from halogen and hydrogen; and
wherein at least two of R₁, R₂, and R₃ are halogen;
b) optionally a dehalogenation reagent;
c) a reducing agent; and
d) a solvent; and
ii) reacting the mixture.

2. The method of claim 1, wherein the compound comprising a metal is selected from a Grignard reagent and a lithium-containing compound.

3. The method of claim 2, wherein the Grignard reagent is selected from MeMgCl, iPrMgCl, *i*PrMgBr, EtMgCl, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof.

4. The method of claim 3, wherein the Grignard reagent is *i*Pr₂NMgCl.

5. The method of claim 2, wherein the lithium-containing compound is selected from LDA, nBuLi, and combinations thereof.

6. The method of claim 1, wherein the solvent C) in the mixture formed in step I) is selected from THF, toluene, 1,4-dioxane, Me-THF, and combinations thereof.

7. The method of claim 6, wherein the solvent is THF.

8. The method of claim 1, wherein the carbonyl-containing compound is selected from dimethylcarbonate, N,N-dimethylacetamide, carbon dioxide, and combinations thereof.

9. The method of claim 8, wherein the carbonyl-containing compound is carbon dioxide.

10. The method of any of claims 1-9, wherein the inorganic base is selected from powder sodium hydroxide, powder potassium hydroxide, potassium carbonate, potassium phosphate, powder sodium methoxide, powder potassium t-butoxide, and combinations thereof.

11. The method of any of claims 1-10, wherein the solvent used when mixing the compound of Formula II with the compound of Formula IV in the presence of an inorganic base and optionally an additive is selected from toluene, N,N-dimethylformamide, N,N-dimethylacetamide, N-methyl-2-pyrrolidone, acetonitrile, and combinations thereof.

12. The method of any of claims 1-11, wherein the additive used when mixing the compound of Formula II with the compound of Formula IV in a solvent in the presence of an inorganic base is selected from potassium iodide, a phase transfer catalyst, and combinations thereof.

## Patentansprüche

1. Verfahren zur Herstellung einer Verbindung der Formel VI 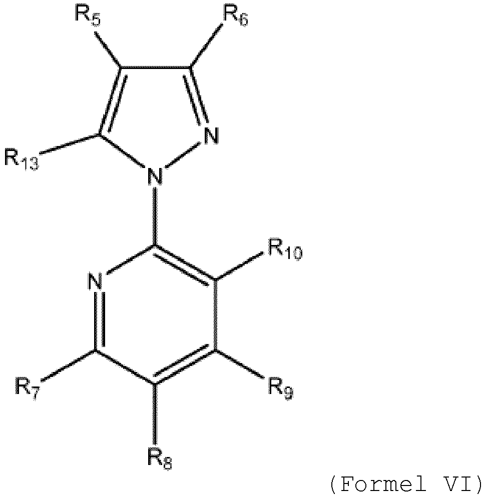
wobei R₅ - R₁₀ jeweils unabhängig aus Wasserstoff und Halogen ausgewählt sind und
R₁₃ für eine organische Säure steht, wobei das Verfahren Folgendes umfasst:
I) Bilden einer Mischung, umfassend
A) eine Verbindung der Formel III 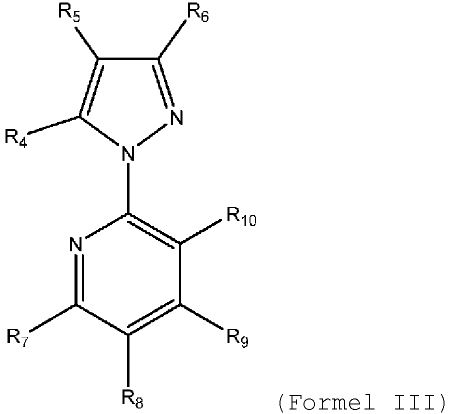
wobei R₄ für Wasserstoff steht und
R₅ - R₁₀ jeweils unabhängig aus Wasserstoff und Halogen ausgewählt sind;
B) eine carbonylgruppenhaltige Verbindung;
C) ein Lösungsmittel;
D) eine Verbindung, die ein Metall umfasst; und
E) gegebenenfalls ein Additiv; und
II) Umsetzen der Mischung; und
wobei die Verbindung der Formel III durch Mischen einer Verbindung der Formel II mit einer Verbindung der Formel IV in einem Lösungsmittel in Gegenwart einer anorganischen Base und gegebenenfalls eines Additivs hergestellt wird;
wobei die Verbindung der Formel II die folgende Struktur aufweist: 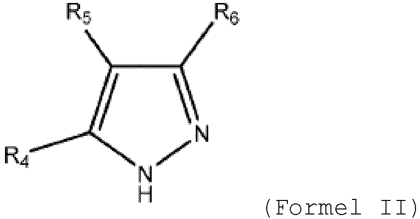
wobei R₄, R₅ und R₆ jeweils unabhängig aus Wasserstoff und Halogen ausgewählt sind und
wobei mindestens eines von R₄, R₅ und R₆ für Wasserstoff steht;
wobei die Verbindung der Formel IV die folgende Struktur aufweist: 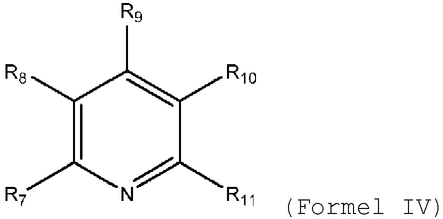
wobei R₇ - R₁₁ jeweils unabhängig aus Wasserstoff und Halogen ausgewählt sind; und
wobei die Verbindung der Formel II gebildet wird durch
i) Bilden einer Mischung, umfassend
a) eine oder mehrere Verbindungen der Formel I 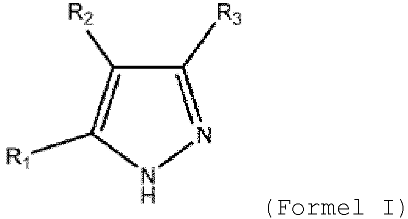
wobei R₁, R₂ und R₃ jeweils unabhängig aus Halogen und Wasserstoff ausgewählt sind und
wobei mindestens zwei von R₁, R₂ und R₃ für Halogen stehen;
b) gegebenenfalls ein Dehalogenierungsmittel;
c) ein Reduktionsmittel und
d) ein Lösungsmittel; und
ii) Umsetzen der Mischung.

2. Verfahren nach Anspruch 1, wobei die Verbindung, die ein Metall umfasst, aus einem Grignard-Reagenz und einer lithiumhaltigen Verbindung ausgewählt wird.

3. Verfahren nach Anspruch 2, wobei das Grignard-Reagenz aus MeMgCl, iPrMgCl, iPrMgBr, EtMgCl, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl und Kombinationen davon ausgewählt wird.

4. Verfahren nach Anspruch 3, wobei es sich bei dem Grignard-Reagenz um *i*Pr₂NMgCl handelt.

5. Verfahren nach Anspruch 2, wobei die lithiumhaltige Verbindung aus LDA, nBuLi und Kombinationen davon ausgewählt wird.

6. Verfahren nach Anspruch 1, wobei das Lösungsmittel (C) in der in Schritt I gebildeten Mischung aus THF, Toluol, 1,4-Dioxan, Me-THF und Kombinationen davon ausgewählt wird.

7. Verfahren nach Anspruch 6, wobei es sich bei dem Lösungsmittel um THF handelt.

8. Verfahren nach Anspruch 1, bei die carbonylgruppenhaltige Verbindung aus Dimethylcarbonat, N,N-Dimethylacetamid, Kohlendioxid und Kombinationen davon ausgewählt wird.

9. Verfahren nach Anspruch 8, wobei es sich bei der carbonylgruppenhaltigen Verbindung um Kohlendioxid handelt.

10. Verfahren nach einem der Ansprüche 1-9, wobei die anorganische Base aus pulverförmigem Natriumhydroxid, pulverförmigem Kaliumhydroxid, Kaliumcarbonat, Kaliumphosphat, pulverförmigem Natriummethoxid, pulverförmigem Kalium-t-butoxid und Kombinationen davon ausgewählt wird.

11. Verfahren nach einem der Ansprüche 1-10, wobei das beim Mischen der Verbindung der Formel II mit der Verbindung der Formel IV in Gegenwart einer anorganischen Base und gegebenenfalls eines Additivs verwendete Lösungsmittel aus Toluol, N,N-Dimethylformamid, N,N-Dimethylacetamid, N-Methyl-2-pyrrolidon, Acetonitril und Kombinationen davon ausgewählt wird.

12. Verfahren nach einem der Ansprüche 1-11, wobei das beim Mischen der Verbindung der Formel II mit der Verbindung der Formel IV in einem Lösungsmittel in Gegenwart einer anorganischen Base verwendete Additiv aus Kaliumiodid, einem Phasentransferkatalysator und Kombinationen davon ausgewählt wird.

## Revendications

1. Procédé de préparation d'un composé de formule VI 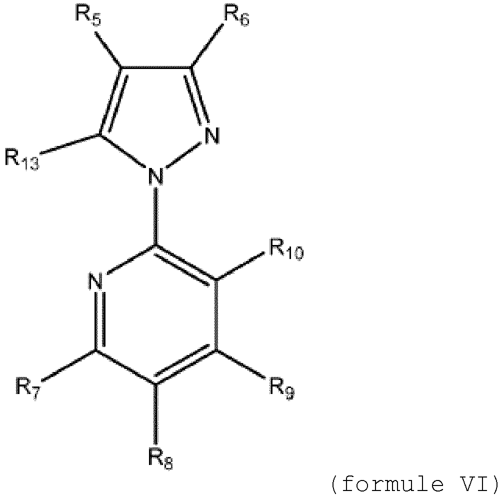
dans laquelle chacun parmi R₅ à R₁₀ est indépendamment choisi parmi hydrogène et halogène ; et
R₁₃ est un acide organique, le procédé comprenant
I) la formation d'un mélange comprenant
A) un composé de formule III 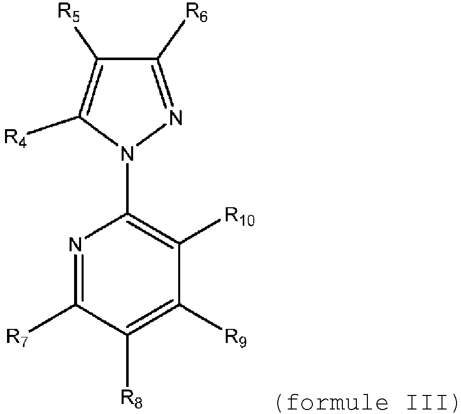
dans laquelle R₄ est hydrogène ; et
chacun parmi R₅ à R₁₀ est indépendamment choisi parmi hydrogène et halogène ;
B) un composé contenant un carbonyle ;
C) un solvant ;
D) un composé comprenant un métal ; et
E) éventuellement un additif ; et
II) la mise en réaction du mélange ; et
dans lequel le composé de formule III est préparé en mélangeant un composé de formule II avec un composé de formule IV dans un solvant en présence d'une base inorganique et éventuellement d'un additif ;
dans lequel le composé de formule II a la structure : 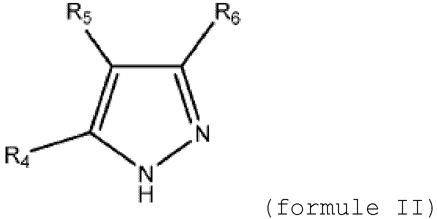
dans laquelle chacun parmi R₄, R₅ et R₆ est indépendamment choisi parmi hydrogène et halogène ; et
dans laquelle au moins l'un parmi R₄, R₅ et R₆ est hydrogène ;
dans laquelle le composé de formule IV a la structure : 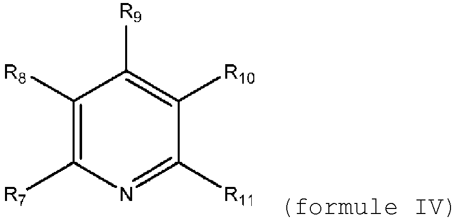
dans laquelle chacun parmi R₇ à R₁₁ est indépendamment choisi parmi hydrogène et halogène ; et
dans laquelle le composé de formule II est formé par
i) la formation d'un mélange comprenant
a) un ou plusieurs composés de formule I 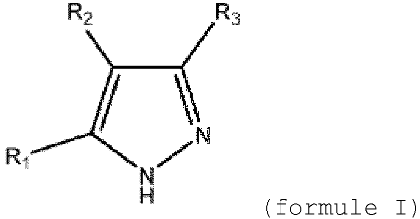
dans laquelle chacun parmi R₁, R₂ et R₃ est indépendamment choisi parmi halogène et hydrogène ; et
dans laquelle au moins deux parmi R₁, R₂ et R₃ sont halogène ;
b) éventuellement un réactif de déshalogénation ;
c) un agent réducteur ; et
d) un solvant ; et
ii) la mise en réaction du mélange.

2. Procédé selon la revendication 1, dans lequel le composé comprenant un métal est choisi parmi un réactif de Grignard et un composé contenant du lithium.

3. Procédé selon la revendication 2, dans lequel le réactif de Grignard est choisi parmi MeMgCl, *i*PrMgCl, iPrMgBr, EtMgCl, *i*Pr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl et leurs combinaisons.

4. Procédé selon la revendication 3, dans lequel le réactif de Grignard est *i*Pr₂NMgCl.

5. Procédé selon la revendication 2, dans lequel le composé contenant du lithium est choisi parmi LDA, nBuLi et leurs combinaisons.

6. Procédé selon la revendication 1, dans lequel le solvant C) dans le mélange formé dans l'étape I) est choisi parmi le THF, le toluène, le 1,4-dioxane, le Me-THF et leurs combinaisons.

7. Procédé selon la revendication 6, dans lequel le solvant est le THF.

8. Procédé selon la revendication 1, dans lequel le composé contenant un carbonyle est choisi parmi le carbonate de diméthyle, le N,N-diméthylacétamide, le dioxyde de carbone et leurs combinaisons.

9. Procédé selon la revendication 8, dans lequel le composé contenant un carbonyle est le dioxyde de carbone.

10. Procédé selon l'une quelconque des revendications 1 à 9, dans lequel la base inorganique est choisie parmi l'hydroxyde de sodium en poudre, l'hydroxyde de potassium en poudre, le carbonate de potassium, le phosphate de potassium, le méthoxyde de sodium en poudre, le t-butoxyde de potassium en poudre et leurs combinaisons.

11. Procédé selon l'une quelconque des revendications 1 à 10, dans lequel le solvant utilisé lors du mélange du composé de formule II avec le composé de formule IV en présence d'une base inorganique et éventuellement d'un additif est choisi parmi le toluène, le N,N-diméthylformamide, le N,N-diméthylacétamide, la N-méthyl-2-pyrrolidone, l'acétonitrile, et leurs combinaisons.

12. Procédé selon l'une quelconque des revendications 1 à 11, dans lequel l'additif utilisé lors du mélange du composé de formule II avec le composé de formule IV dans un solvant en présence d'une base inorganique est choisi parmi l'iodure de potassium, un catalyseur de transfert de phase et leurs combinaisons.
